# EUROPEAN PATENT APPLICATION

(11) **EP 4 032 515 A2**
(43) Date of publication of application: **27.07.2022**
(21) Application number: 20865366.7
(22) Date of filing: 18.09.2020
(51) Int. Cl.: A61F 9/06

(54) **WELDING PROTECTIVE EQUIPMENT WITH OPTICAL FUNCTIONAL LAYER AND PANEL CONTROL TECHNOLOGY APPLIED THERETO**

(30) Priority: 20.09.2019 KR 20190116359
(71) Applicant: Otos Wing Co., Ltd., Seoul 08521 (KR)
(72) Inventor: HUH, Sung Won, Seoul 06084 (KR)
(74) Representative: Hoffmann Eitle
(86) International application number: PCT/KR2020/012599
(87) International publication number: WO 2021/054753

(57) **Abstract**

The present disclosure relates to a welding protector including an LCD panel. In detail, the present disclosure relates to a welding protector including: a panel unit including a panel for blocking welding light; a first functional member arranged in a front surface direction of the panel; and a second functional member arranged in a rear surface direction of the panel, wherein at least two coating layers arranged on at least one of spaces between the front portion of the first functional member and the rear portion of the second functional member are deposited.

## Description

### TECHNICAL FIELD

The present disclosure relates to a welding protector including an optical functional layer and a method of controlling a panel unit included in the welding protector.

### BACKGROUND ART

Welding generally refers to bonding of two metal materials by locally heating and melting the metal using fusibility of metal. Because light of high heat and high luminance and a gas are generated during a welding process, a worker wears a welding protector as a kind of protectors.

A welding protector is used to protect the face and eyeballs of a worker performing an operation such as a welding, a cutting, etc. In addition, such a welding protector includes an anti-glare device (hereinafter, referred to as a 'cartridge') installed therein, in order to protect eyes of a worker against intense harmful light generated when an operation such as welding, cutting, etc. is performed.

Such a cartridge blocks rays of light of 780 nm to 365 nm (for example, ultraviolet rays, infrared rays, etc.), and controls transmission amount of visible ray.

There is a limitation in improving work visibility during an operation while protecting eyes of a worker by controlling the welding protector or the cartridge.

### DESCRIPTION OF EMBODIMENTS

### TECHNICAL PROBLEM

The present disclosure may provide a welding protector capable of improving stability of a worker and a work efficiency, and a method of controlling an LCD panel in a welding protector.

### TECHNICAL SOLUTION

According to an embodiment of the present disclosure, a welding protector includes: a panel unit including a panel for shielding welding light; a first functional member arranged to face a front surface direction of the panel, the front surface direction facing the welding light; and a second functional member arranged to face a rear surface direction that is opposite to the front surface direction of the panel, wherein at least two optical functional layers are arranged between a region of the first functional member facing the front surface direction and a region of the second functional member facing the rear surface direction.

The optical functional layers may include an infrared ray blocking material, an ultraviolet ray blocking material, or a reflection reducing material.

The optical functional layers may be formed on the region of the first function member facing the front surface, the region of the first functional member facing the rear surface, the region of the second functional member facing the front surface, and the region of the second functional member facing the rear surface.

The welding protector may include an infrared ray absorbent adhesive arranged on at least one region in the space between the first functional member and the second functional member.

The panel unit may include a plurality of panels, and the welding protector may include: a sensor for sensing the welding light; and a processor for determining a total shading degree of the cartridge unit based on welding light information obtained through the sensor and controlling shading degrees of the plurality of panels based on the determined shading degree.

Other aspects, features and advantages of the disclosure will become better understood through the accompanying drawings, the claims and the detailed description.

### ADVANTAGEOUS EFFECTS OF THE DISCLOSURE

According to a welding protector and a method of controlling an LCD panel in a welding protector of the present disclosure, stability of a worker and work efficiency may be improved.

### BRIEF DESCRIPTION OF THE DRAWINGS

FIG. 1 is a diagram showing a welding protector according to an embodiment of the present disclosure.
FIG. 2 is a schematic block diagram for describing components of a welding protector, for controlling a cartridge unit according to an embodiment of the present disclosure.
FIG. 3A and FIG. 3B are exploded perspective views of a cartridge unit according to an embodiment of the present disclosure.
FIG. 4A to FIG. 4D are diagrams for describing embodiments, in which a bandpass coating layer according to an embodiment of the present disclosure is deposited.
FIG. 5A to FIG. 5H are diagrams for describing embodiments, in which an infrared ray (IR) coating layer according to an embodiment of the present disclosure is deposited.
FIG. 6A and FIG. 6B are diagrams for describing various examples of a cartridge unit according to an embodiment of the present disclosure.
FIG. 7 to FIG. 9 are flowcharts describing a method of controlling a plurality of LCD panels, according to an embodiment of the present disclosure.
FIG. 10 is a graph of a shading control voltage with respect to each LCD panel, when a panel unit according to an embodiment of the present disclosure includes two LCD panels.
FIG. 11 is a graph of a shading control voltage with respect to each LCD panel, when a panel unit according to an embodiment of the present disclosure includes three LCD panels.
FIG. 12 is a graph of shading control voltages with respect to a plurality of LCD panels, when there is a user command with respect to a shading degree according to an embodiment of the present disclosure.

### BEST MODE

According to an embodiment of the present disclosure, a welding protector includes: a panel unit including a panel for shielding welding light; a first functional member arranged to face a front surface direction of the panel, the front surface direction facing the welding light; and a second functional member arranged to face a rear surface direction that is opposite to the front surface direction of the panel, wherein at least two optical functional layers are arranged between a region of the first functional member facing the front surface direction and a region of the second functional member facing the rear surface direction.

### MODE OF DISCLOSURE

Hereinafter, one or more embodiments of the present disclosure will be described below with reference to accompanying drawings. A variety of modifications may be made to the present disclosure and there are various embodiments of the disclosure, and particular embodiments will be illustrated in the drawings and described in detail in the written description. However, it is not intended to limit the various embodiments of the present disclosure to particular embodiments, and it is to be appreciated that all changes, equivalents, and substitutes that do not depart from the spirit and technical scope are encompassed in the present disclosure. Regarding the description of the drawings, like reference numerals may be used for like components.

In the present specification, it is to be understood that the terms such as "including," "having," and "comprising" are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added.

In various embodiments of the present disclosure, the expression "or" includes any or all combinations of words enumerated together. For example, the expression "A or B" may include A, may include B, or may include both A and B.

In the present disclosure, expressions including ordinal numbers, such as "first" and "second," etc., may modify various elements. However, such elements are not limited by the above expressions. For example, the above expressions do not limit the sequence and/or importance of the elements. The above expressions are used merely for the purpose of distinguishing an element from the other elements. For example, a first element could be termed a second element, and similarly, a second element could be also termed a first element without departing from the scope of the present disclosure.

In the case where an element is referred to as being "connected" or "accessed" to other elements, it should be understood that not only the element is directly connected or accessed to the other elements, but also another element may exist between them. Contrarily, when an element is referred to as being "directly coupled" or "directly connected" to any other element, it should be understood that no element is interposed therebetween.

In an embodiment of the present disclosure, terms such as "module", "unit", "part", etc. are used to refer to components that perform at least one function or operation, and such components may be implemented in the form of hardware or software or may be implemented in a combination of hardware and software. In addition, a plurality of "modules", "units", "parts", etc. may be integrated as at least one module or chip and implemented as at least one processor, except for a case in which each of the modules, the units, and the parts needs to be implemented as individual specific hardware.

Such terms as those defined in a generally used dictionary are to be interpreted to have the meanings equal to the contextual meanings in the relevant field of art, and are not to be interpreted to have ideal or excessively formal meanings unless clearly defined in the present disclosure.

Hereinafter, one or more embodiments of the disclosure will be described in detail with reference to accompanying drawings.

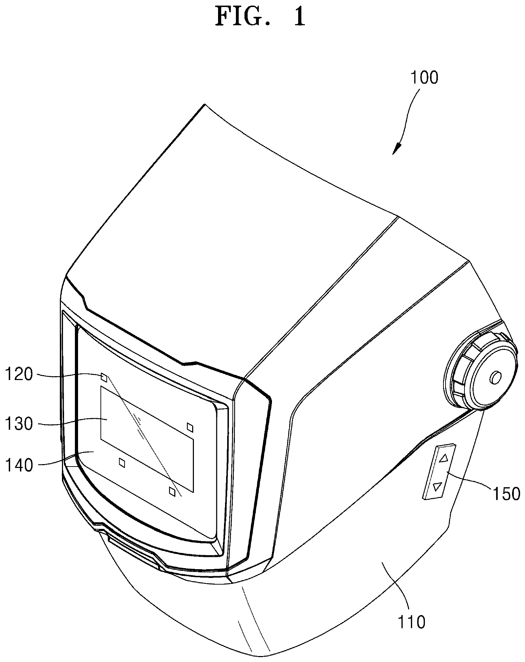

FIG. 1 is a diagram showing a welding protector according to an embodiment of the present disclosure.

A welding protector 100 according to an embodiment is to protect a worker during a welding operation, and may include a main body 110, a sensor 120, a cartridge unit 130, a housing unit 140, and a user interface unit 150.

The main body 110 forms an outer appearance of the welding protector 100, and may prevent sparks generated during the welding operation from flying toward the face of the worker, thereby preventing a safety accident that may occur to the worker.

The main body 110 may include various materials, for example, the main body 110 may include at least one selected from ABS (styrene resin including styrene, acrylonitrile, and butadiene), polycarbonate (PC, thermoplastic resin prepared by reacting bisphenol A with phosgene, etc.), and PVC (plastic having vinyl chloride as a main component), but materials therefor are not limited thereto.

The sensor 120 may sense welding light from a welding surface on which working is being performed. When a welding operation, a cutting operation, etc. is performed, light may be generated, and the sensor 120 may sense the light. For example, the sensor 120 may sense an illuminance of the light. In detail, the sensor 140 may include an illuminance sensor or a photosensor, but is not limited thereto.

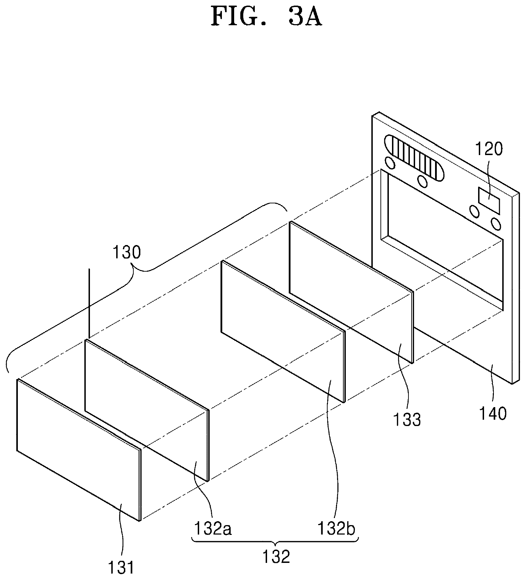

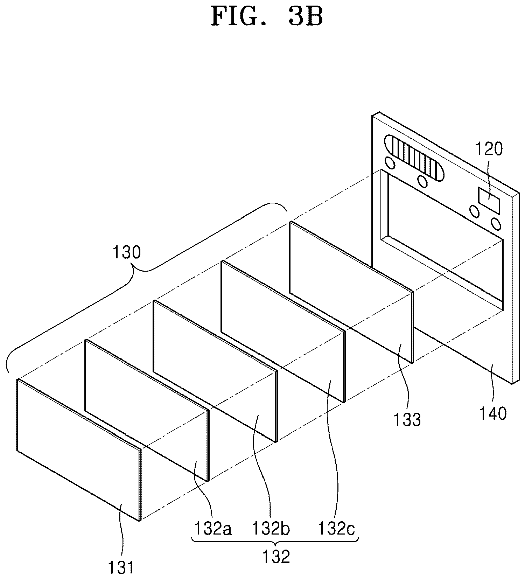

The cartridge unit 130 is an element for protecting eyes of the worker against intense harmful light generated when the welding operation, a cutting operation, etc. is performed. Referring to FIGS. 3A and 3B, the cartridge unit 130 may include a first functional member 131, a panel unit 132, and a second functional member 133.

The first functional member 131 and the second functional member 133 are disposed respectively on a front surface and a rear surface of the panel unit 132, and an additional filter layer may be coated and deposited to block harmful light such as ultraviolet (UV) ray and infrared (IR) ray.

Here, the first functional member 131 and the second functional member 133 may be realized by using a transparent material (e.g., glass), but are not limited thereto, and according to an embodiment, the first functional member 131 and the second functional member 133 may include IR absorbing glass. That is, the first functional member 131 and the second functional member 133 may function as optical functional layers, like a bandpass coating layer and an IR coating layer. Here, the optical functional layer denotes a functional layer for blocking IR ray, for blocking UV ray, or reducing reflection.

The panel unit 132 may include at least two liquid crystal display (LCD) panels, and the LCD panels may each have a shading degree that may change to change a light transmittance of electromagnetic wave such as a visible ray. Here, the shading degrees of the plurality of LCD panels may be adjusted respectively according to separate shading degree control signals, and this will be described in detail later.

The user interface unit 150 receives an input of a user command from a user. The user may set a shading degree of the cartridge unit 130 through the user interface unit 150. For example, when the user presses a button for raising shading degree (up arrow button) via the user interface unit 150, the panel unit 132 included in the cartridge unit 130 is blackened to raise the shading degree. Although FIG. 1 illustrates that the user interface unit 150 is implemented as buttons, this is an example. That is, the user interface unit 150 may be implemented by any means capable of inputting a command for increasing/decreasing the shading degree.

The housing unit 140 of the present disclosure is connected to the main body 110 to form an exterior of the cartridge unit 130, and the sensor 120 may be mounted therein.

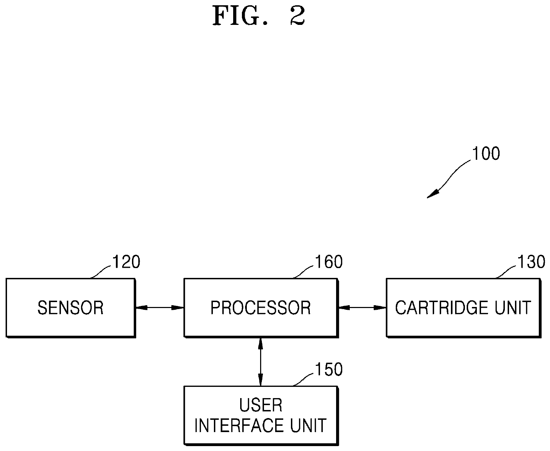

FIG. 2 is a schematic block diagram for describing components of the welding protector, for controlling a cartridge unit according to an embodiment of the present disclosure.

Referring to FIG. 2, the welding protector 100 may further include a processor 160 in addition to the sensor 120, the cartridge unit 130, and the user interface unit 150. Descriptions about the sensor 120, the cartridge 130, and the user interface 150 that are the same as those provided with reference to FIG. 1 will be omitted.

The sensor 120 according to an embodiment of the present disclosure may sense welding light or welding ray generated at a site of the welding operation, and the processor 160 may acquire welding light information through the sensor 120.

The processor 160 may determine a shading degree to be applied to the cartridge unit 130 based on the obtained welding light information. Here, the shading degree may be determined in a plurality of levels. For example, the shading degree may be divided into a level 1 to a level 13, and as the shading degree level increases, the shading degree may increase, and the amount of electromagnetic waves (e.g., visible rays) transmitted through the cartridge unit 130 may decrease.

The processor 160 according to an embodiment of the present disclosure may control the shading degree by blackening the plurality of LCD panels included in the cartridge unit 130. The processor 160 may control the shading degree by controlling signals for the plurality of LCD panels.

For example, the processor 160 may simultaneously control the plurality of LCD panels, and in another example, may simultaneously control at least two LCD panels.

According to an alternative embodiment, the processor 160 may accurately adjust the shading degree level by inputting an independent shading control signal to each of the LCD panels.

In detail, the processor 160 may determine a shading degree level to be applied to the cartridge unit 130 based on welding light information obtained through the sensor 120, and when the determined shading degree level is equal to or lower than a predetermined first reference level, the processor 160 may output a first shading control signal corresponding to the determined shading degree level to control the shading degree level of the first LCD panel from among the plurality of LCD panels. Here, the first LCD panel may be blackened in response to the first shading control signal, thereby controlling the shading degree to correspond to the determined shading degree level.

When the shading degree determined based on the welding light information exceeds the predetermined first reference level, the processor 160 may output to the cartridge unit 130 the first shading control signal and the second shading control signal corresponding to the determined shading degree level. Here, among the plurality of LCD panels of the cartridge unit 130, the first LCD panel is blackened in response to the first shading control signal, and the second LCD panel is blackened in response to the second shading control signal, thereby controlling the shading degree to correspond to the determined shading degree level.

According to another embodiment of the present disclosure, when the determined shading degree level exceeds the preset first reference level and equal to or less than the preset second reference level, the processor 160 may control the shading degrees of the first LCD panel and the second LCD panel, and when the determined shading degree level exceeds the preset second reference level, the processor 160 may control the shading degrees of the first LCD panel, the second LCD panel, and the third LCD panel.

Here, the preset first reference level may be the shading degree level 8 and the preset second reference level may be the shading degree level 11, but this is merely an example, and the first reference level and the second reference level may be variously set according to embodiments.

According to another embodiment of the present disclosure, the plurality of LCD panels may include four or more LCD panels, and accordingly, the preset reference level may be also changed. For example, when there are four or more LCD panels, the processor 160 may control the shading degree using only the first LCD panel in a case in which the shading degree level to be applied to the cartridge unit 130 is 4 or less, may control the shading degree using the first LCD panel and the second LCD panel in a case in which the shading degree level is 4 to level 8, may control the shading degree using the first LCD panel to the third LCD panel in a case in which the shading degree level is 9 to level 11, and may control the shading degree using the first LCD panel to the fourth LCD panel in a case in which the shading degree level is 11 to level 13.

As such, the welding protector of the present embodiment may easily realize a desired shading degree by individually controlling a desired LCD panel from among the plurality of LCD panels. Precise shading characteristics corresponding to various welding light can be secured.

In addition, power consumption may be reduced without affecting shading characteristics through individual control of the plurality of LCD panels.

In addition, in an alternative embodiment, the processor 160 may apply to the cartridge unit 130 a shading degree level corresponding to a user command input through the user interface unit 150. According to an embodiment of the present disclosure, the processor 160 may apply to the cartridge unit 130 a shading degree level corresponding to a user command, over a shading degree level determined based on the welding light information obtained through the sensor 120.

In an alternative embodiment, when the shading degree level determined by using the welding light information acquired through the sensor 120 exceeds a limit value, the processor 160 may apply to the cartridge unit 130 the shading degree level determined by using the welding light information over the shading degree level corresponding to the user command.

As such, according to the present disclosure, the user may be allowed to see the working site through a desired shading degree level, and at the same time, the shading degree is automatically increased when the shading degree level exceeds the limit value of the shading degree level that is determined to be harmful to the eyes of the user (e.g., level 12), and accordingly high user experience and stability may be secured.

In addition, the processor 160 of the present disclosure may control overall operations of the welding protector 100 by using various programs stored on a memory (not shown). For example, the processor 160 may include a CPU, a RAM, a ROM, and a system bus. Here, the ROM is a configuration for storing a set of instructions for system booting, and the CPU copies an operating system stored on the memory of the welding protector 100 according to the instructions stored on the ROM and executes the operating system to boot the system. When the booting is completed, the CPU may copy various applications stored on the memory to the RAM, execute the applications, and perform various operations. Although it is described that the processor 160 includes only one CPU, the processor 160 may be implemented to include a plurality of CPUs (or DSP, SoC, etc.).

According to an embodiment of the present disclosure, the processor 160 may be implemented as a digital signal processor (DSP), a microprocessor, or a time controller (TCON) for processing a digital signal. However, the present disclosure is not limited thereto, and the processor may include one or more selected from a central processing unit (CPU), a micro controller unit (MCU), a micro processing unit (MPU), a controller, an application processor (AP), a communication processor (CP), and an ARM processor, or may be defined as a corresponding term. In addition, the processor 160 may be implemented as a System on Chip (SoC) or a large-scale integration (LSI) in which a processing algorithm is embedded, or may be implemented in the form of a field programmable gate array (FPGA).

The processor 160 may be mounted on the welding protector 100 and electrically connected to the sensor 120, the cartridge unit 130, and the user interface unit 150. According to an embodiment of the present disclosure, the processor 160 may be implemented as a separate device from the welding protector 100. In this case, the welding protector 100 may further include a communication unit (not shown), and may transmit a signal corresponding to the welding light information acquired by using the sensor 120 to the processor 160 through the communication unit (not shown). Likewise, the communication unit (not shown) may transmit a signal corresponding to the user command input through the user interface unit 150 to the processor 160. The processor 160 may transmit to a communication unit (not shown) a signal corresponding to welding light information and a shading control signal generated based on a signal corresponding to an input user command to control a shading degree of the cartridge unit 130.

Here, the communication unit (not shown) may include a module which is capable of performing short-range wireless communication (e.g., Bluetooth, Wifi, Wifi-Direct) or long-range wireless communication (3G, High-Speed Downlink Packet Access (HSDPA) or Long Term Evolution (LTE)).

FIG. 3A and FIG. 3B are exploded perspective views of a cartridge unit according to the embodiment of the present disclosure.

Referring to FIG. 3A, the cartridge unit 130 according to the embodiment of the present disclosure may include the first functional member 131, the panel unit 132, and the second functional member 133.

The first functional member 131 may be formed to block at least a part of harmful light including ultraviolet rays and infrared rays generated during a welding operation. That is, the first functional member 131 may be formed to at least partially block the ultraviolet rays generated in a welding operation, and in an alternative embodiment, the first functional member 131 may be formed to at least partially block the infrared rays and the ultraviolet rays generated during a welding operation.

The first functional member 131 may be disposed at a position farthest from the face of the worker. The first functional member 131 may be disposed adjacent to a front surface direction of a first LCD panel 132a in the panel unit 132. In this case, the front surface direction may be a direction toward a point where the welding operation is performed in a direction away from the face of the worker.

The panel unit 132 may include one or more LCD panels 132a and 132b, and may selectively block the visible rays generated during a welding operation. The first and second LCD panels 132a and 132b may control the shading degrees by blackening in response to the shading control signal from the processor 160.

Although FIG. 3A illustrates that the panel unit 132 is implemented by the first and second LCD panels 132a and 132b, the present disclosure is not limited thereto, and the panel unit 132 may include three LCD panels as shown in FIG. 3B. The panel unit may be modified variously according to embodiments, e.g., may include four or more LCD panels. As shown in FIG. 3B, as the number of LCD panels increases, the welding protector 100 of the present disclosure may accurately realize the shading degree to be implemented with low power.

The panel unit 132 according to the embodiment of the present disclosure may receive power from a power supply unit (not shown) installed in the housing unit 140 and may visually display operation information on at least one of a plurality of LCD panels 132a to 132c constituting the panel unit 132. Here, the LCD panel displaying the operation information may receive an electrical signal and visually display the operation information related to the welding operation.

The second functional member 133 may be disposed at a position between the panel unit 132 and the face of the worker.

The second functional member 133 may be directly or indirectly coupled to the panel unit 132 to reduce or prevent damage to the panel unit 132. The second functional member 133 may be disposed adjacent to a rear surface direction of the panel unit 132. In this case, the rear surface direction may be a direction opposite to the front surface direction and directed toward the face of the worker.

The first functional member 131 and the second functional member 133 of the present disclosure may include a transparent material such as glass. A bandpass coating layer, an IR coating layer, or an anti-reflection (AR) coating layer may be formed on at least one surface of the first functional member 131 or the second functional member 133.

Infrared rays and ultraviolet rays in the welding light generated during the welding operation may be absorbed or reflected by a bandpass coating layer and an infrared (IR) coating layer of the first functional member 131 and the second functional member 133. In addition, a reflectivity may be reduced and a transmittance of the electromagnetic wave may be increased by an anti-reflection (AR) coating layer. That is, a high ratio of visible rays may be transmitted to the eyes of the user due to the bandpass coating layer, the infrared (IR) coating layer, or the anti-reflection (AR) coating layer.

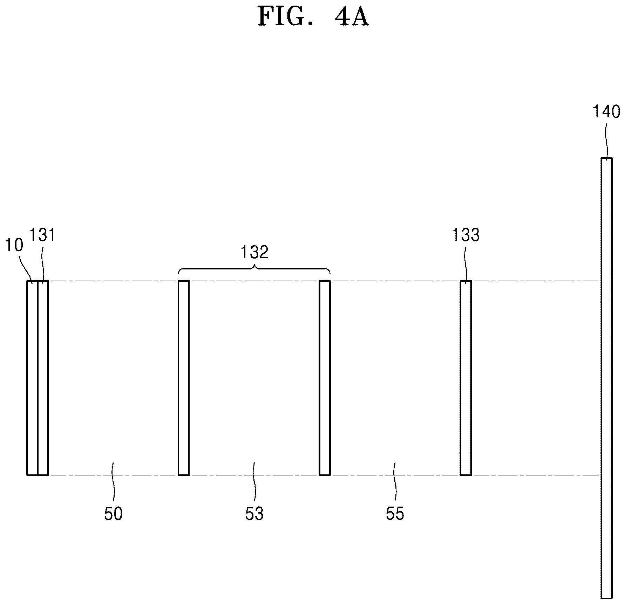

The bandpass coating layer, the infrared (IR) coating layer, or the anti-reflection (AR) coating layer will be described in detail with reference to FIGS. 4A to 6B.

FIG. 4A to FIG. 6B are exploded plan views of the cartridge unit according to the embodiment of the present disclosure.

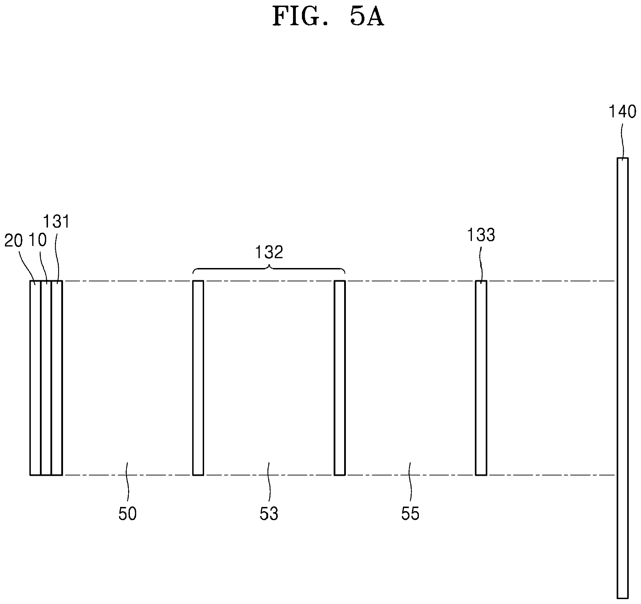

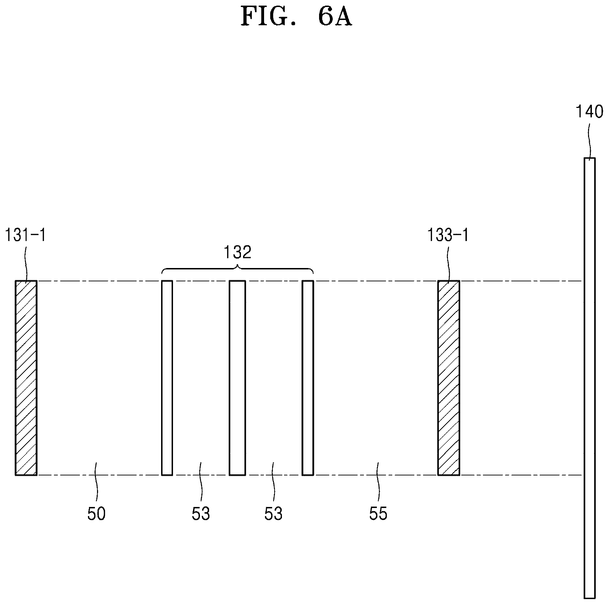

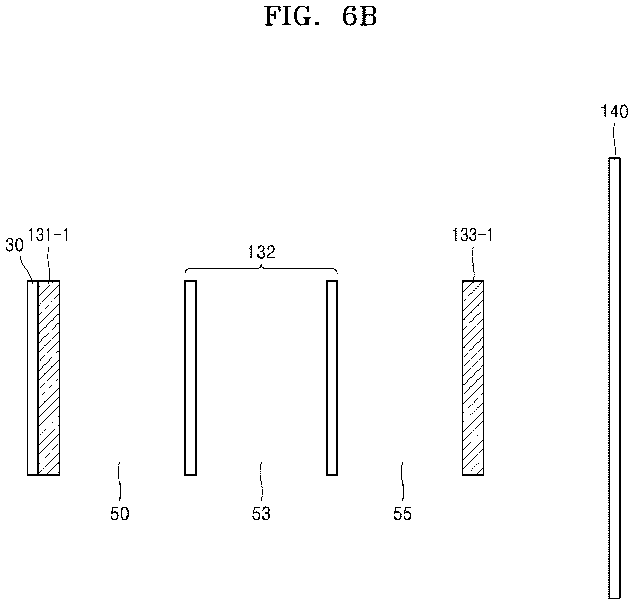

In particular, FIGS. 4A to 4D are diagrams for describing embodiments in which the bandpass coating layer of the present disclosure is deposited, FIGS. 5A to 5H are diagrams for describing embodiments in which the infrared (IR) coating layer of the present disclosure is deposited, and FIGS. 6A and 6B are diagrams for describing various embodiments of the cartridge unit.

The front surface portion described in FIGS. 4A to 6B refers to a direction in which a welding operation is performed (a direction away from the housing unit 140), and the rear surface portion refers to a direction toward the user's eyes (a direction toward the housing unit 140).

Referring to FIG. 4A, a first bandpass coating layer 10 may be deposited on the front surface portion of the first functional member 131 of the welding protector 100 of the present disclosure. In the present embodiment, 100% of a paint for blocking the UV ray may be deposited on the front surface portion of the first functional member 131.

The first bandpass coating layer 10 or an optical bandpass filter layer selectively transmits a part of a spectrum and reduces or blocks transmission of a certain wavelength. In particular, the first bandpass coating layer 10 of the present disclosure may reduce and block transmission of ultraviolet ray and infrared ray spectra and may selectively transmit only the visible ray spectrum area.

The first bandpass coating layer 10 may include a silver film having a spectral reflection characteristic. The silver thin film may absorb light in an ultraviolet ray wavelength area, but may reflect light of a different wavelength band, and may selectively transmit a visible ray area by blocking light in an infrared ray area from the reflected light using a prism.

According to an alternative embodiment, transmission characteristics of the silver thin film may be appropriately controlled by adjusting a thickness of the silver thin film, thereby controlling the spectrum transmission for light of a certain wavelength area including the visible ray.

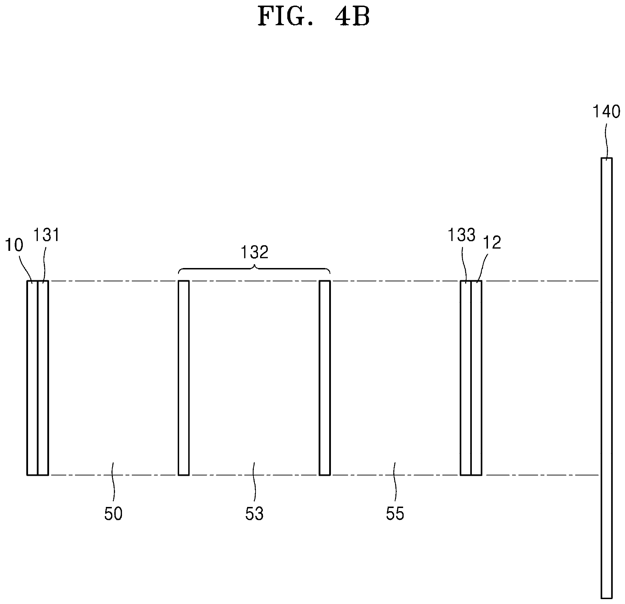

Referring to FIG. 4B, the first bandpass coating layer 10 may be deposited on the front surface portion of the first functional member 131 of the welding protector 100, and a third bandpass coating layer 12 may be deposited on the rear surface portion of the second functional member 133.

According to the embodiment of the present disclosure, the first bandpass coating layer 10 may be only deposited to 50% of a thin film for blocking a certain spectrum, and the third bandpass coating layer 12 may be deposited to the remaining 50%. However, it is an example, and the ratio may be variously changed, for example, the first bandpass coating layer 10 may be deposited to 40% and the third bandpass coating layer 12 may be deposited to 60%.

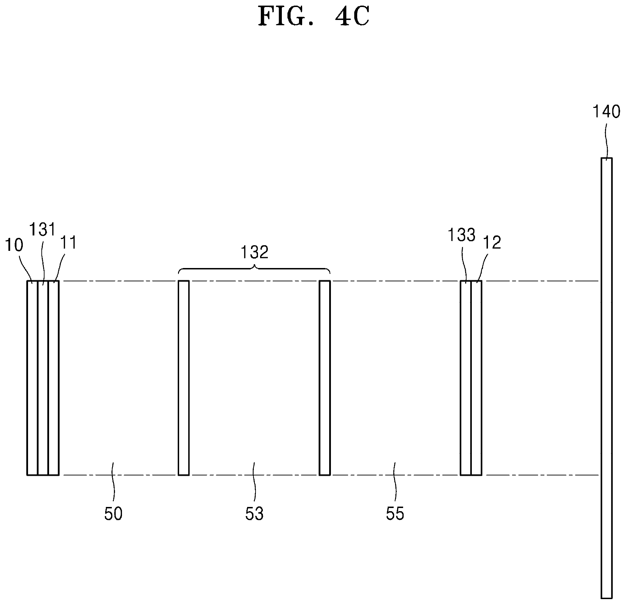

According to the embodiment of FIG. 4B, the transmittance of the electromagnetic wave is increased, as compared with the case where 100% of the bandpass coating layer 10 is deposited on the first functional member 131. Referring to FIG. 4C, the first functional member 131 may include the first bandpass coating layer 10 and a second bandpass coating layer 11 formed on opposite sides, e.g., the front and rear surface portions thereof. Here, a UV paint may be dispersed in each of the first bandpass coating layer 10, the second bandpass coating layer 11, and the third bandpass coating layer 12, and then may be applied. For example, the first band-pass coating layer 10 may be deposited to 40%, and the second bandpass coating layer 11 and the third bandpass coating layer 12 may be each deposited to 30%, but embodiments of the present disclosure are not limited thereto.

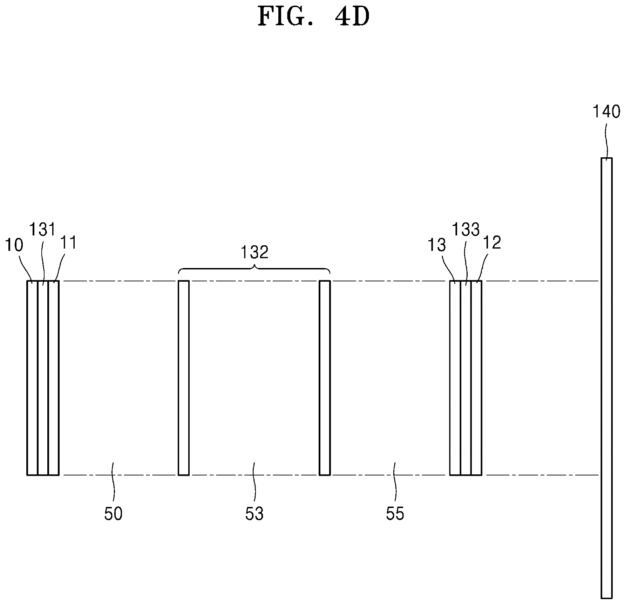

In addition, in another embodiment, referring to FIG. 4D, the third bandpass coating layer 12 and a fourth bandpass coating layer 13 may be formed on the front surface portion and the rear surface portion of the second functional member 133, respectively. Here, the bandpass coating layer may be dispersed and deposited respectively as the first bandpass coating layer 10, the second bandpass coating layer 11, the third bandpass coating layer 12, and the fourth bandpass coating layer 13. For example, the first bandpass coating layer 10 may be deposited to 40%, and the second bandpass coating layer 11, the third bandpass coating layer 12, and the fourth bandpass coating layer 13 may be each deposited to 20%, but the embodiments of the present disclosure are not limited thereto.

The welding protector 100 according to the present embodiment may reduce or prevent reflection of welding light by depositing the bandpass coating layers on one surface of the first functional member 131 and another surface facing the above surface.

In addition, in an alternative embodiment, the welding protector 100 may reduce or prevent reflection of welding light by depositing the bandpass coating layers on one surface of the second functional member 133 and another surface facing the above surface.

That is, as shown in FIGS. 4C and 4D, the welding protector 100 according to the present disclosure performs a coating on opposite surfaces of the functional member, thereby preventing reflection of the electromagnetic wave.

In addition, the welding protector 100 according to the embodiment of the present disclosure may include an adhesive in which an IR absorbent is added in at least one of intermediate regions 50, 53, and 55 that are among the first functional member 131, the panel unit 132, and the second functional member 133, in order to block additional IR ray. Here, the adhesive may include an optical clear resin (OCR) having transparency and adhesiveness, but is not limited thereto.

According to the embodiment of the present disclosure, because the IR absorbent is included in at least one of the regions 50, 53, and 55, the IR ray may be blocked without forming an additional IR coating layer. However, the present disclosure is not limited thereto, and the IR absorbent may be included in the intermediate regions 50, 53, and 55, and moreover, the IR coating layer may be added to the first functional member or the second functional member to improve the function.

In addition, the first functional member 131 and the second functional member 133 according to the embodiment of the present disclosure may include insulation glass that absorbs the infrared (IR) ray and/or the ultraviolet (UV) ray. The insulation glass may include sodium calcium silicate alkali glass, sodium calcium silicon glass, or a blue glass composition (Fe₂O₃: 0.4%, MnO₂: 0.15%, CoO: 0.005-0.025%, TiO₂: 0-1%, reducing agent: anthracite, etc.). In addition, the insulation glass composition may contain, but is not limited to, 65-80% of SiO₂, 0-5% of Al₂O₃, 0-5% of B₂O₃, 5-15% of CaO, 0-2% of MgO, 9-18% of Na₂O, 0-10% of K₂O, 0-5% of BaO, 0.7-1.6% of Fe₂O₃, 0.1-1.2% of CeO, and 0-1.5% of TiO₂.

That is, in the welding protector 100 according to the present disclosure, the bandpass coating layer is deposited on the front surface portions and/or the rear surface portions of the first functional member 131 and the second functional member 133 that are implemented by insulation glass absorbing the IR ray and/or UV ray, thereby filtering the IR ray and the UV ray.

FIG. 5A to FIG. 5H are diagrams for describing an embodiment, in which the infrared (IR) coating layer is deposited according to the present disclosure.

Referring to FIG. 5A, the first functional member 131 may include a first IR coating layer 20 on the front surface portion of the first bandpass coating layer 10. In the present embodiment, 100% of a paint for blocking IR ray may be deposited on the front surface portion of the first functional member 131.

Here, the IR coating may be performed by using a diamond-type carbon (DLC) layer, or may be performed by using an IR component capable of being soldered in a sealed manner. In addition, the present disclosure is not limited thereto, and the IR optical coating may be applied to the first functional member 131 by using a preform filter, a window, and a cover plate. In addition, the paint for the IR coating may include cesium tungsten oxide, a binder resin, an ultraviolet treatment agent, an ester-based solvent, and an organic solvent, but is not limited thereto.

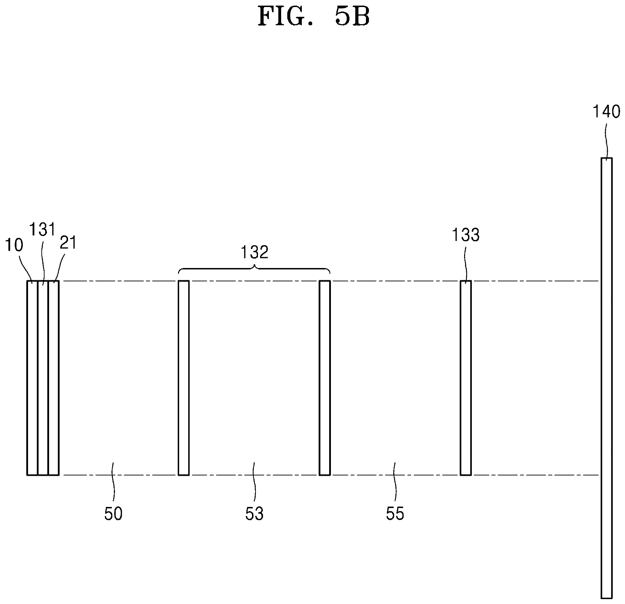

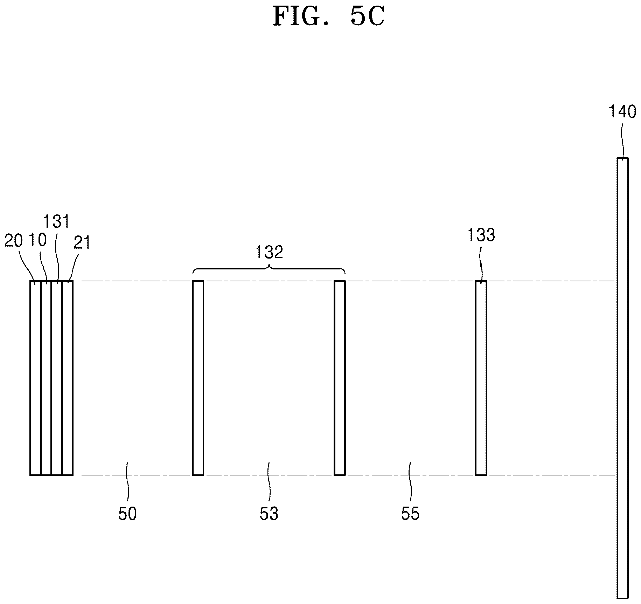

Referring to FIG. 5B, the first functional member 131 may include the first bandpass coating layer 10 on the front surface portion thereof and a second IR coating layer 21 on the rear surface portion thereof. However, the above is merely an example, and the first functional member 131 of the present disclosure may include the first IR coating layer 20 on the front surface portion and the second IR coating layer 21 on the rear surface portion thereof as shown in FIG. 5C. The first IR coating layer 20 may be deposited on the front surface portion of the first bandpass coating layer 10. As such, the present disclosure has an effect of effectively suppressing light reflection while blocking both UV ray and IR ray.

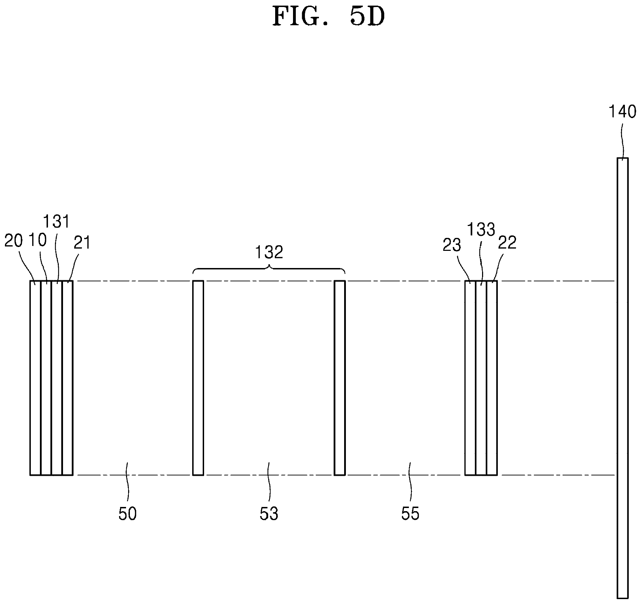

Although FIG. 5C shows that the bandpass coating layer and the IR coating layer are formed on the front and rear surface portions of the first functional member 131, this is merely an example. That is, as shown in FIG. 5D, a third IR coating layer 22 and a fourth IR coating layer 23 may be formed on the front and rear surface portions of the second functional member 133 as well as the first functional member 131. Here, the IR paint may be dispersed in each of the first IR coating layer 20, the second IR coating layer 21, the third IR coating layer 22, and the fourth IR coating layer 23 and then may be applied. For example, the first IR coating layer 20 may include 40% of paint, and the second IR coating layer 21, the third IR coating layer 22, and the fourth IR coating layer 23 may respectively include 20% of paint, but the present disclosure is not limited thereto.

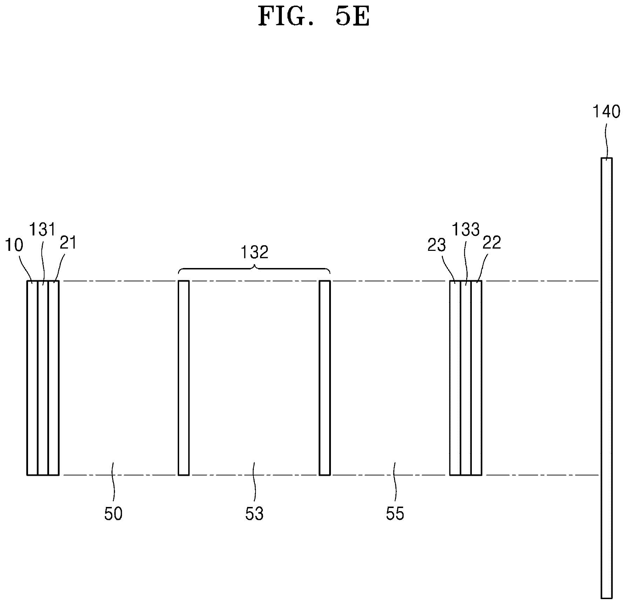

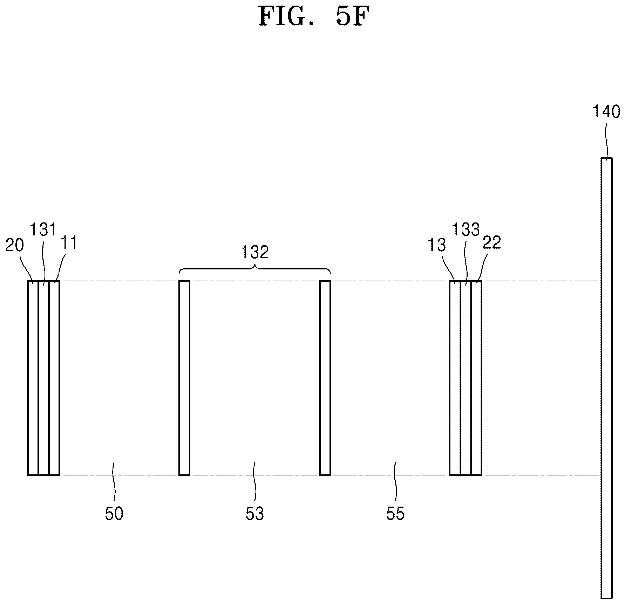

Meanwhile, the IR coating layer and the bandpass coating layer may be mixed and deposited as a single layer respectively on the front and rear surface portions of the functional member in the welding protector 100 according to the present disclosure. For example, referring to FIG. 5E, the first functional member 131 may have the first bandpass coating layer 10 deposited on the front surface portion thereof and the second IR coating layer 21 deposited on the rear surface portion thereof. The second functional member 133 may have a third IR coating layer 23 deposited on the front surface portion thereof and the third bandpass coating layer 12 deposited on the rear surface portion thereof. Also, referring to FIG. 5F, the first functional member 131 may have the first IR coating layer 20 deposited on the front surface portion thereof and the second bandpass coating layer 11 deposited on the rear surface portion thereof. The second functional member 133 may have a third bandpass coating layer 12 deposited on the front surface portion thereof and the third IR coating layer 22 deposited on the rear surface portion thereof.

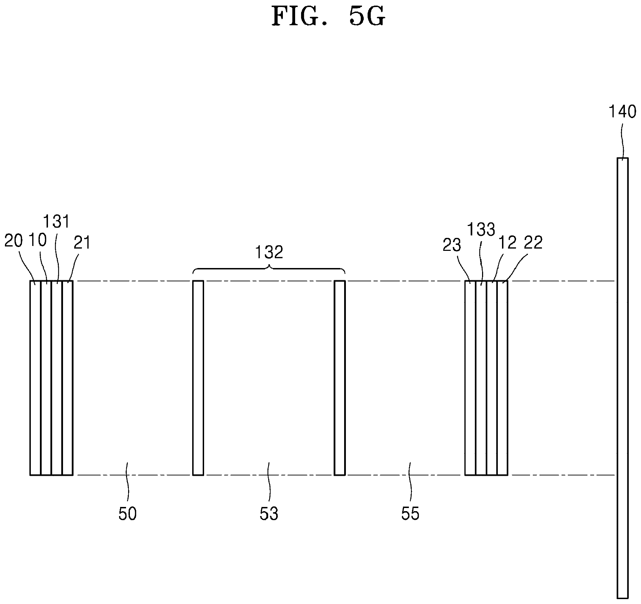

The IR coating layer and the bandpass coating layer may be mixed and deposited as one or more layers respectively on the front and rear surface portions of the functional member in the welding protector 100 according to the present disclosure. For example, referring to FIG. 5G, the first functional member 131 may have the first bandpass coating layer 10 deposited on the front surface portion thereof, and the first IR coating layer 20 may be deposited on the front surface portion of the first bandpass coating layer 10, and the second IR coating layer 21 may be deposited on the rear surface portion of the first bandpass coating layer 10. In addition, the fourth IR coating layer 23 may be deposited on the front surface portion of the second functional member 133, and the third bandpass coating layer 12 may be deposited on the rear surface portion of the second functional member 133. Moreover, the third IR coating layer 22 may be deposited on the rear surface portion of the third bandpass coating layer 12.

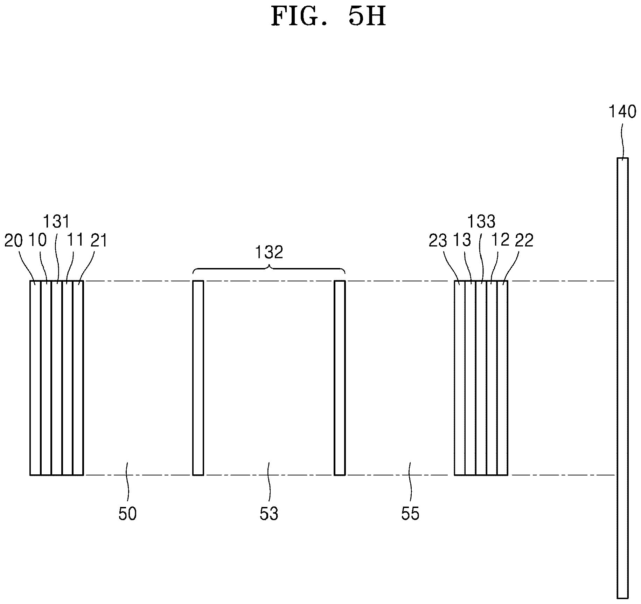

Referring to FIG. 5H, the second bandpass coating layer 11 may be deposited on the rear surface portion of the first functional member 131, and the second IR coating layer 21 may be deposited on the rear surface portion of the second bandpass coating layer 11. Likewise, the fourth bandpass coating layer 13 may be deposited on the front surface portion of the second functional member 133, the fourth IR coating layer 23 may be deposited on the front surface portion of the fourth bandpass coating layer 13, the third bandpass coating layer 12 may be deposited on the rear surface portion of the second functional member 133, and the third IR coating layer 22 may be deposited on the rear surface portion of the third bandpass coating layer 12.

Although FIGS. 5A to 5H illustrate the embodiments in which the IR coating layer and the bandpass coating layer are mixed and deposited, the coating layer may be deposited in various combinations. In addition, the paint may be variously coated according to the embodiments such that the sum of the paint included in the plurality of IR coating layers is to be 100%, and the plurality of bandpass coating layers may also be variously deposited according to the embodiments such that the sum is to be 100%.

FIG. 6A and FIG. 6B are plan views of the cartridge unit according to an embodiment of the present disclosure.

Referring to FIG. 6A, the cartridge unit 130 may include the panel unit 132 including three LCD panels while including a first multi-layer coated functional member 131-1 and a second multi-layer coated functional member 133-1. Here, the first multi-layer coated functional member 131-1 and the second multi-layer coated functional member 133-1 refer to functional members having the IR coating layers and/or the bandpass coating layers variously combined and deposited on the front portions and/or the rear portions thereof as shown in FIGS. 4A to 5H. For example, in the first multi-layer coated functional member 131-1, the IR coating layer and/or the bandpass coating layer may be deposited only on the front surface portion or the rear surface portion, or the IR coating layer and/or the bandpass coating layer may be deposited on both the front surface portion and the rear surface portion.

According to an embodiment of the present disclosure, an optical adhesive including a functional paint such as an IR absorbent may be applied to the intermediate regions 50, 53, and 55 among the first multi-layer coated functional member 131-1, the second multi-layer coated functional member 133-1, and the three LCD panels.

Referring to FIG. 6B, in the cartridge unit 300 of the present disclosure, an anti-reflection (AR) coating layer 30 may be deposited on the front surface portion of the first multi-layer coated functional member 131-1. The AR coating layer 30 may be a coating layer that is deposited on a substrate such as glass, PC, PMMA, etc. using the principle of optical interference to reduce a reflectivity and increase a transmittance. For example, when not being coated with AR, about 4% of incident light is reflected at each surface. However, when the AR coating layer is present, the surface reflection is reduced to 0.1% or less. The AR coating layer may include a combination of thin dielectric multilayer films formed of a material such as oxide, metal, rare earth elements, etc. The number of layers, a thickness of each layer, and a refractive index between layers may be variously determined in the AR coating layer. In addition, the AR coating layer 30 of the present disclosure may be generally formed by alternately depositing a dielectric material and a metal material such as tantalum pentoxide (Ta₂O₅) or aluminum oxide (Al₂O₃) on a thin layer, but the present disclosure is not limited thereto.

The AR coating layer 30 may be deposited to be specified to a certain incident angle and polarization state of the welding light, e.g., s-polarized light, p-polarized light, random polarized light, etc., and the performance of the AR coating may be deteriorated at other angles and polarization states than the specified incident angle and polarization state. Accordingly, as shown in FIG. 6B, the AR coating layer 30 may be deposited on the front surface portion of the first multi-layer coated functional member 131-1 corresponding to the outermost portion of the cartridge unit 130. However, according to embodiments, the AR coating layer 30 may be disposed on at least one region between a region facing the front surface of the first multi-layer coated functional member 131-1 and a region facing the rear surface of the second multi-layer coated functional member, as well as the front portion of the first multi-layer coated functional member 131-1.

In addition, in the above case, the welding protector 100 may also include an adhesive in which an IR absorbent is added in at least one of the intermediate regions 50, 53, and 55 among the first multi-layer coated functional member 131-1, the second multi-layer coated functional member 133-1, and the three LCD panels. Here, the adhesive may include, but is not limited to, an optical clear resin (OCR) having transparency and adhesiveness.

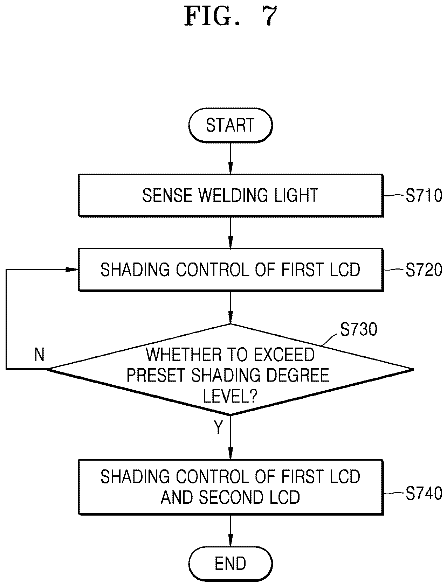

FIG. 7 to FIG. 9 are flowcharts illustrating a method of controlling a plurality of LCD panels according to an embodiment of the present disclosure.

FIG. 7 illustrates a method of controlling an LCD panel in an embodiment, in which there are two LCD panels in the panel unit 132. The welding protector 100 of the present disclosure may sense welding light via the sensor 120 (S710), and may control a shading degree of the first LCD panel based on the determined shading degree with respect to the visible ray (S720).

After that, the welding protector 100 may determine whether the determined shading degree exceeds a preset shading degree level (S730). When it is determined that the determined shading degree exceeds the preset shading degree (S730-Y), the welding protector 100 controls the shading degree by using the first LCD panel and the second LCD panel (S740), and when it is determined that the determined shading degree is equal to or less than the preset shading degree (S730-N), the welding protector 100 may control the shading degree by using the first LCD panel (S730). When the shading degree is controlled by using the first LCD panel and the second LCD panel, the welding protector 100 may perform the shading degree control of the panel unit 132 flexibly through independent shading control signals with respect to the first LCD panel and the second LCD panel.

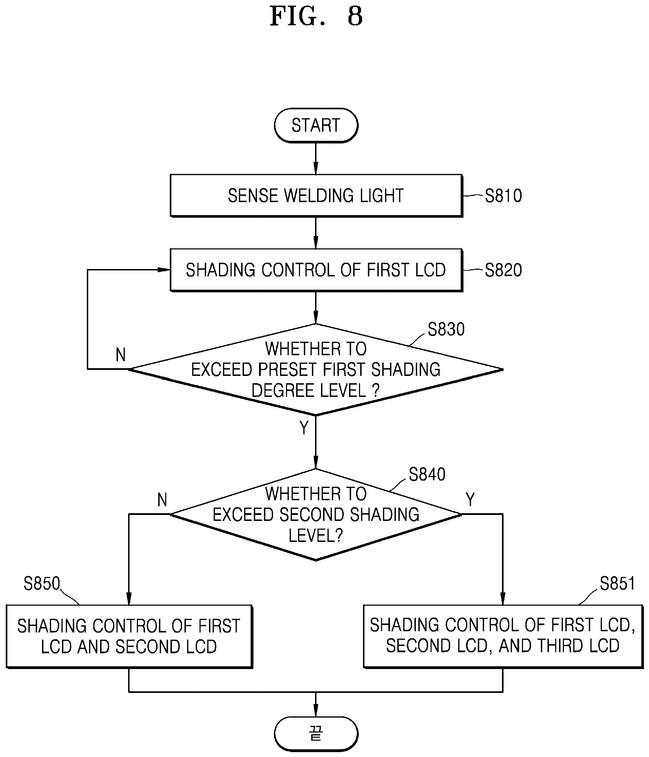

In particular, FIG. 8 is a flowchart illustrating a method of controlling a panel in an embodiment in which there are three LCD panels in the panel unit 132. The welding protector 100 of the present disclosure may sense welding light via the sensor 120 (S810), and may control a shading degree of the first LCD panel based on the determined shading degree with respect to the visible ray (S820). After that, the welding protector 100 may determine whether the determined shading degree exceeds a preset first shading degree level (S830).

When it is determined that the determined shading degree exceeds the preset first shading degree level (S830-Y), the welding protector 100 may control the shading degree by using the first LCD panel and the second LCD panel (S850) in a case in which the determined shading degree does not exceed the preset second shading degree level (S840-N), and may control the shading degree by using all of the first to third LCD panels (S851) in a case in which the determined shading degree exceeds the preset second shading degree (S840-Y).

Here, the reference shading degree level such as the first shading degree level (e.g., level 8) and the second shading degree level (e.g., level 11) may be flexibly changed according to various embodiments. In addition, the reference shading degree level may be input to the processor 160 at the time of shipment, but may be changed by the user.

When the shading degree is controlled by using at least two of the first to third LCD panels, the welding protector 100 may perform the shading degree control of the panel unit 132 flexibly via independent shading control signals with respect to the LCD panels.

However, the embodiment of FIGS. 7 and 8 is merely an example, and the welding protector 100 may control the shading degree of the plurality of LCD panels according to various embodiments.

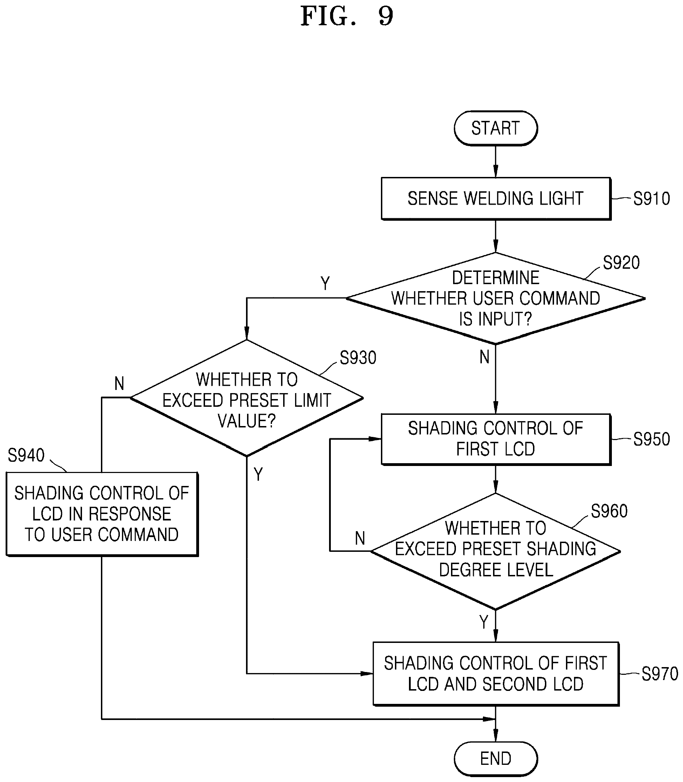

For example, referring to FIG. 9, when the welding light is sensed by the sensor 120 (S910), the welding protector 100 according to the present disclosure may determine whether a user command is input (S920).

The user command may be input through the user interface unit 150. For example, the welding protector 100 may control the panel unit 132 to correspond to the shading degree set by the user, regardless of the shading degree determined based on the welding light information.

When there is no user command input (S920-N), the welding protector 100 may control the shading degree of the first LCD panel based on the shading degree determined with respect to the visible ray (S950). After that, the welding protector 100 may determine whether the determined shading degree exceeds a preset shading degree level (S960). When it is determined that the determined shading degree exceeds the preset shading degree (S960-Y), the welding protector 100 controls the shading degree by using the first LCD panel and the second LCD panel (S970), and when it is determined that the determined shading degree is equal to or less than the preset shading degree (S960-N), the welding protector 100 may control the shading degree by using the first LCD panel (S950). When the shading degree is controlled by using the first LCD panel and the second LCD panel, the welding protector 100 may perform the shading degree control of the panel unit 132 flexibly through independent shading control signals with respect to the first LCD panel and the second LCD panel.

On the other hand, when there is an input of a user command (S920-Y), the welding protector 100 may control the shading degree by using the first and second LCD panels (S970) in a case in which the shading degree level that may be determined based on the welding light information sensed by the sensor 120 exceeds the preset limit value (S930-Y). This is to protect the user's eyes when the shading degree set by the user is relatively low even though it is determined that welding light which may be harmful for the eyes of the user is generated. That is, the welding protector 100 may automatically provide the shading degree corresponding to the shading degree level determined by using the first LCD panel and the second LCD panel, thereby protecting the user's eyes.

When the shading degree level that may be determined based on the welding light information sensed by the sensor 120 does not exceed the preset limit value (S930-N), the welding protector 100 may control the shading of the LCD panel in response to a user command (S940).

As such, according to the present disclosure, the stability may be secured by protecting the eyes of the user while improving the user experience such that the user may control the shading degree to be the desired level.

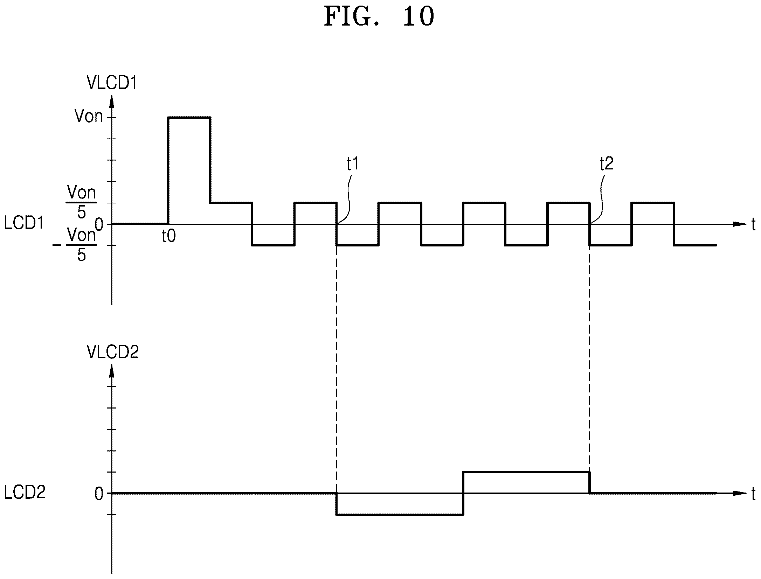

FIG. 10 to FIG. 12 are graphs of a shading control voltage for each of LCD panels in a panel unit including a plurality of LCD panels, in a welding protector according to an embodiment of the present disclosure.

In particular, FIG. 10 shows a graph of a shading control voltage for each LCD panel when the panel unit 132 includes two LCD panels. Referring to FIG. 10, an x-axis denotes time t and a y-axis denotes shading control voltages VLCD1 and VLCD2 with respect to an LCD1 (hereinafter, a first LCD panel) and an LCD2 (hereinafter, a second LCD panel) included in the panel unit 132.

When welding light is incident at time t0, the processor 160 may apply a high shading control voltage Von to the first LCD to prevent instantaneous glare. The welding protector 100 according to the embodiment of the present disclosure may control the shading degree using only the first LCD when the determined shading degree is equal to or less than the preset first level, and may control the shading degree using both the first LCD and the second LCD when the determined shading degree exceeds the preset first level.

That is, in FIG. 10, the processor 160 of the present disclosure outputs the shading control signal (or the shading control voltage) only to the first LCD panel in order to realize the shading degree equal to or less than the preset first level from the time t0 to time t1, and outputs an independent shading control signal (or shading control voltage) to each of the first LCD panel and the second LCD panel in order to realize the shading degree exceeding the preset first level from the time t1 to time t2. After the time t2, in order to realize the shading degree that is equal to or less than the preset first level again, the processor 160 may adjust the shading degree by outputting the shading control signal (or the shading control voltage) only to the first LCD panel.

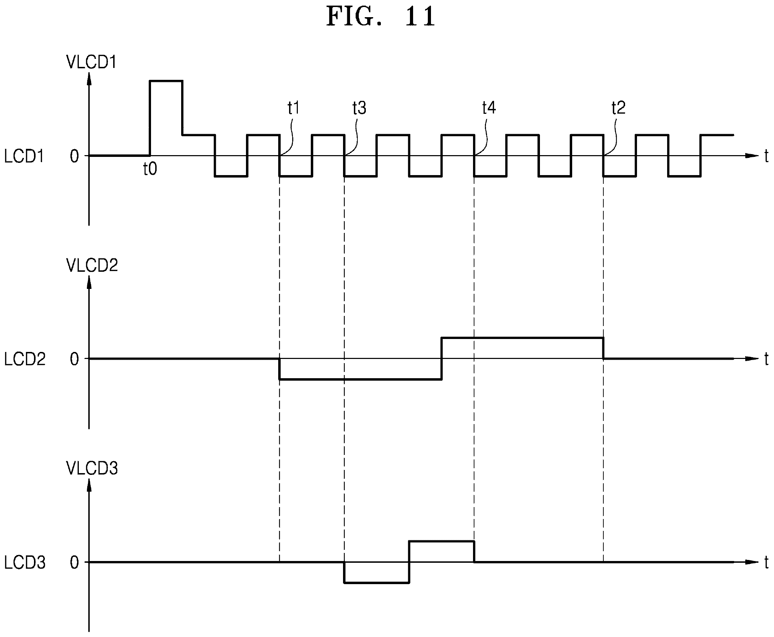

FIG. 11 shows a graph of a shading control voltage for each LCD panel when the panel unit 132 includes three LCD panels. Referring to FIG. 11, an x-axis denotes time t, and a y-axis represents shading control voltages VLCD1, VLCD2, and VLCD3 for the LCD1 (hereinafter, referred to as a first LCD), the LCD2 (hereinafter, referred to as a second LCD), and an LCD3 (hereinafter, referred to as a third LCD) included in the panel unit 132.

When welding light is incident at time t0, the processor 160 applies a high shading control voltage Von to the first LCD to prevent instantaneous glare. The welding protector 100 according to the embodiment of the present disclosure may control the shading degree by using only the first LCD when the determined shading degree is equal to or less than the preset first level, may control the shading degree using the first LCD and the second LCD when the determined shading degree is greater than the preset first level and equal to or less than the preset second level, and may control the shading degree by using all of the first LCD to the third LCD when the determined shading degree is greater than the preset second level.

The processor 160 of the present disclosure outputs a shading control signal (or a shading control voltage) only to the first LCD to realize the shading degree that is equal to or less than the preset first level from time t0 to time t1, and outputs an independent shading signal (or a shading control voltage) to each of the first LCD panel and the second LCD panel to realize the shading degree that exceeds the preset first level and equal to or less than the preset second level from the time t1 to time t3 and from time t4 to time t2, thereby controlling the shading degree.

In addition, in order to realize the shading degree exceeding the preset second level from the time t3 to the time t4, a shading control signal (or a shading control voltage) may be output for each of the first LCD, the second LCD, and the third LCD to control the total shading degree of the panel unit 132. After the time t2, in order to realize the shading degree that is equal to or less than the preset first level, the processor 160 outputs the shading control signal (or the shading control voltage) only to the first LCD to control the shading degree.

The welding protector 100 of the present disclosure applies an independent shading control voltage to each LCD panel from among the plurality of LCD panels, and adjusts the voltage applied to the LCD panels according to the shading level (for example, adjusts a frequency of the voltages applied to the second LCD and the third LCD), thereby accurately realizing the shading degree.

In addition, the welding protector 100 of the present disclosure has an effect of reducing unnecessary power consumption in that the number of driving LCDs is flexibly controlled according to the change in the state of welding light.

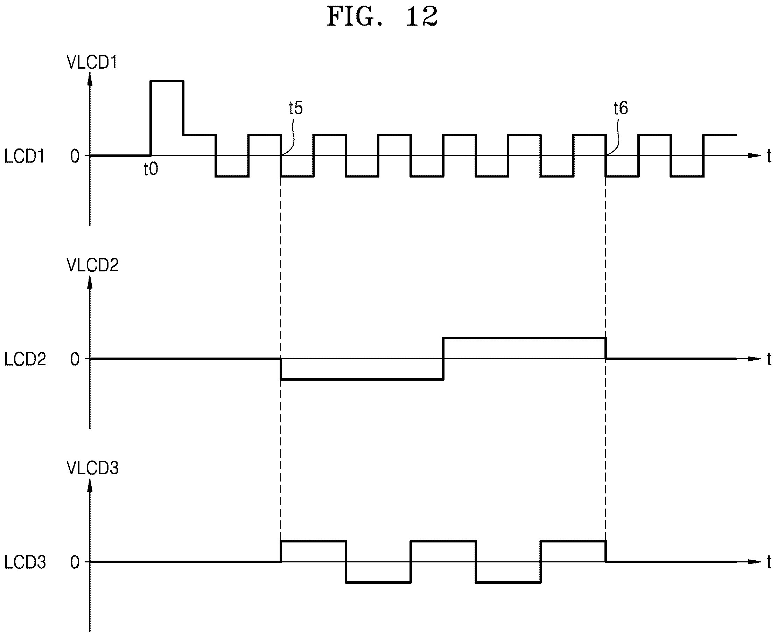

FIG. 12 is a graph showing shading control voltages with respect to a plurality of LCD panels, when there is a user command with respect to a shading degree, according to an embodiment of the present disclosure.

Referring to FIG. 12, an x-axis denotes time t, and a y-axis represents shading control voltages VLCD1, VLCD2, and VLCD3 for the LCD1 (hereinafter, referred to as a first LCD), the LCD2 (hereinafter, referred to as a second LCD), and an LCD3 (hereinafter, referred to as a third LCD) included in the panel unit 132.

When welding light is incident at time t0, the processor 160 applies a high shading control voltage Von to the first LCD to prevent instantaneous glare. The processor 160 may apply a shading control voltage only to the first LCD panel in order to control the light shielding level to correspond to a user command for the shading degree. Meanwhile, when the determined shading degree level exceeds the limit value, the welding protector 100 according to the embodiment of the present disclosure may apply to the cartridge unit 130 the shading degree determined based on the welding light information sensed through the sensor 120 over the user command.

That is, when the shading degree level determined based on the welding light information sensed through the sensor 120 exceeds the limit value from time t5 to time t6, the processor 160 may control the light shielding of the panel unit 132 by outputting the shading control signals (or the shading control voltages) with respect to all the first LCD, the second LCD, and the third LCD in order to realize the light shielding level exceeding the limit value.

While the disclosure has been particularly shown and described with reference to exemplary embodiments thereof, it will be understood by those of ordinary skill in the art that various changes in form and details may be made therein without departing from the spirit and scope as defined by the following claims. Therefore, the scope sought to be protected of the disclosure shall be defined by the appended claims.

## Claims

1. A welding protector comprising:
a panel unit including a panel for shielding welding light;
a first functional member arranged to face a front surface direction of the panel, the front surface direction facing the welding light; and
a second functional member disposed to face a rear surface direction that is opposite to the front surface direction of the panel,
wherein at least two optical functional layers are arranged between a region of the first functional member facing the front surface direction and a region of the second functional member facing the rear surface direction.

2. The welding protector of claim 1, wherein
the at least two optical functional layers include an infrared ray blocking material, an ultraviolet ray blocking material, or a reflection reducing material.

3. The welding protector of claim 1, wherein
the at least two optical functional layers are formed on the region of the first function member facing the front surface direction, the region of the first functional member facing the rear surface direction, the region of the second functional member facing the front surface direction, and the region of the second functional member facing the rear surface direction.

4. The welding protector of claim 1, further comprising
an infrared ray absorbent adhesive arranged on at least one region from among spaces between the first functional member and the second functional member.

5. The welding protector of claim 1, wherein
the panel unit includes a plurality of panels, and
the welding protector further comprises:
a sensor sensing the welding light; and
a processor configured to determine a total shading degree of the cartridge unit based on welding light information obtained through the sensor and control shading degrees of the plurality of panels based on the determined shading degree.
